# EUROPEAN PATENT SPECIFICATION

(11) **EP 3 412 776 B1**
(45) Date of publication and mention of the grant of the patent: **10.04.2024**
(21) Application number: 17174974.0
(22) Date of filing: 08.06.2017
(51) Int. Cl.: C12Q 1/68

(54) **GENETIC POLYMORPHISM ASSOCIATED WITH DOG AFIBRINOGENEMIA**
GENETISCHER POLYMORPHISMUS IM ZUSAMMENHANG MIT HUNDE-AFIBRINOGENÄMIE
POLYMORPHISME GÉNÉTIQUE ASSOCIÉ À L'AFIBRINOGÉNÉMIE DU CHIEN

(43) Date of publication of application: 12.12.2018
(73) Proprietor: Stiftung Tierärztliche Hochschule Hannover, 30559 Hannover (DE)
(72) Inventor: Distl, Ottmar, 30559 Hannover (DE); Metzger, Julia, 30173 Hannover (DE); Mischke, Reinhard, 30966 Hemmingen (DE)
(74) Representative: Grünecker Patent- und Rechtsanwälte PartG mbB

(56) References cited:
- ELLEGREN HANS: "Genomics: the dog has its day.", NATURE 08 DEC 2005, vol. 438, no. 7069, 8 December 2005 (2005-12-08), pages 745-746, XP002772226, ISSN: 1476-4687
- Sree Kanthaswamy: "The author(s) shown below used Federal funds provided by the U.S. Department of Justice and prepared the following final report: Document Title: Development and Validation of a Standardized Canine STR Panel for Use in Forensic Casework", , 1 April 2009 (2009-04-01), XP055391792, Retrieved from the Internet: URL:https://www.ncjrs.gov/pdffiles1/nij/gr ants/226639.pdf [retrieved on 2017-07-18]
- MARGUERITE NEERMAN-ARBEZ ET AL: "Mutations in the fibrinogen gene cluster accounting for congenital afibrinogenemia: an update and report of 10 novel mutations", HUMAN MUTATION, vol. 28, no. 6, 1 June 2007 (2007-06-01), pages 540-553, XP055391605, US ISSN: 1059-7794, DOI: 10.1002/humu.20483
- NEERMAN-ARBEZ MARGUERITE ET AL: "Deletion of the fibrogen alpha-chain gene (FGA) causes congenital afibrogenemia", JOURNAL OF CLINICAL INVESTIGATION, vol. 103, no. 2, January 1999 (1999-01), pages 215-218, XP002772227, ISSN: 0021-9738
- Wilkerson ET AL: "Afibrinogenemia and a circulating antibody against fibrinogen in a Bichon Frise dog", Veterinary Clinical Pathology, vol. 34, no. 2, 1 June 2005 (2005-06-01), pages 148-155, XP93092432, United States ISSN: 0275-6382, DOI: 10.1111/j.1939-165X.2005.tb00029.x

## Description

### TECHNICAL FIELD

The present disclosure relates to a novel single nucleotide polymorphism associated with afibrinogenemia in dogs. The SNP is located in the fibrinogen alpha chain (FGA) gene on canine chromosome 15. The present disclosure encompasses methods and compositions based on the novel genetic polymorphism, as well as corresponding oligonucleotide probes and kits for use in the methods provided.

### BACKGROUND OF THE INVENTION

Genetic testing has been shown to play an increasingly important role in dog breeding. It allows breeders to improve targeted mating in order to avoid genetic diseases in progeny. Nevertheless, intense selection for specific desirable traits and dog types has promoted a large number of genetic defects which have not been investigated so far and are passed on among the dog population without any monitoring possibilities. Especially autosomal recessive mutations have been shown to cause essential problems for several dog breeds as genetic carriers cannot be phenotypically detected without any genetic testing (Metzger *et al.* 2016).

Bleeding disorders represent a large group of diseases which are often not recognized in time before breeding decisions are made, especially due to the varying onsets of the diseases (Brooks 1999b). Various different defects can be found in the dog affecting the platelet activation in the initial phase of hemostasis, like von Willebrand factor changes, or in the secondary hemostasis cascade affecting fibrin formation (Lippi et al. 2015). The absence of coagulation factor I (fibrinogen) is described as hypofibrinogenaemia or afibrinogenemia (Mammen 1976). Typical clinical signs are prolonged clotting time, cord or mucosal bleeding, haematomas, haemarthroses or severe bleeding after trauma or surgery (Lak *et al.* 1999). In human, more than 70 genetic variants have been detected in one of the fibrinogen subunit genes *fibrinogen alpha chain (FGA), fibrinogen beta chain (FGB)* or *fibrinogen gamma chain* (FGG) (Neerman-Arbez *et al.* 2001; Neerman-Arbez & De Moerloose 2007). These fibrinogen deficiencies were found to be inherited as autosomal recessive disorders (De Moerloose *et al.* 2013). In the dog, a hereditary afibrinogenemia was detected in the Bernese Mountain Dog, Bichon Frise and Chihuahua (Kammermann *et al.* 1971; Wilkerson *et al.* 2005; Chambers 2013). In addition, breed dispositions for fibrinogen deficiency were suspected in Borzoi, Collie, Viszla, St. Bernard and mixed breeds, as well as in Domestic Shorthair and Domestic Longhair cats (Brooks 1999a; Smith *et al.* 2005; Wilkerson *et al.* 2005).

Human genome sequencing has shown that genetic variations between individuals are small and amount to <1% of genetic sequences. These DNA sequence variations can take many forms: substitution of single nucleotides, insertion or deletion of single or multiple nucleotides, changes in the number of repetitive sequences, and larger changes in chromosome structure. Most variations involve single-nucleotide change and they are named single nucleotide polymorphisms (SNPs). Genetic markers such as SNPs are preferred types of biomarkers that can be genotyped for diagnosing or assessing the genetic predisposition of various risk factors. Recognizing that the defects resulting from bleeding disorder such as congenital afibrinogenemia produce mild to severe or fatal bleeding diathesis, there is an urgent need for novel genetic markers that are predictive of predisposition to such bleeding disorders.

The present disclosure addresses this need as it allows analyzing a genetic defect causing afibrinogenemia in dogs.

### SUMMARY OF THE INVENTION

It has been recognized by the present inventors that a mutation in the canine fibrinogen alpha chain (FGA) gene on chromosome 15, in particular the mutation NC_006597.3:g.52240694deIA (SEQ ID NO: 1: FGA:g.6296delT (CanFam 3.1), SEQ ID NO: 2: FGA:g.1734deIT (CanFam 3.1)), is a mutation strongly associated with afibrinogenemia in dogs. The SNP mutation is a deletion (delT) in the FGA gene, which causes an alteration of the alpha-subunit of the FGA protein. The SNP mutation provides for a frameshift in the nucleic acid sequence of the FGA gene, resulting in a shortened, mutated, and thus non-functional form of the FGA protein.

The present invention is defined by the appended claims. General aspects of the present disclosure include the following:

### BRIEF DESCRIPTION OF THE DRAWINGS

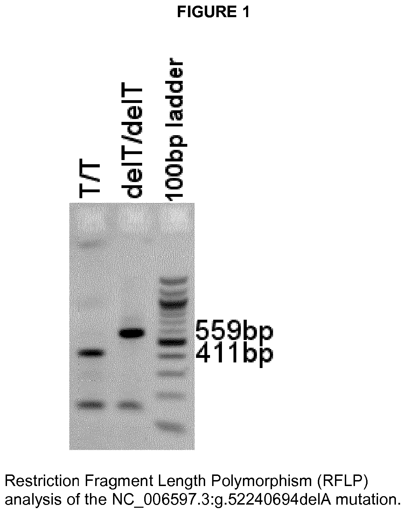

Figure 1 shows Restriction Fragment Length Polymorphism (RFLP) analysis for validation of the NC_006597.3:g.52240694deIA (SEQ ID NO: 1: FGA:g.6296delT, SEQ ID NO: 2. FGA:g.1734delT) mutation. In dogs with the homozygous wild type T/T, a 411 bp fragment is detected whereas homozygous mutant dogs show a 559 bp band.

### DETAILED DESCRIPTION OF THE INVENTION

It has been recognized by the present inventors that a mutation in the canine FGA gene, in particular the mutation NC_006597.3:g.52240694deIA (SEQ ID NO: 1: FGA:g.6296deIT, SEQ ID NO: 2: FGA:g.1734deIT), is a mutation strongly associated with afibrinogenemia in dogs. Specifically, it has been recognized that a homozygous mutant (delT/delT) genotype results in the absence of the coagulation factor I (fibrinogen). The mutation is lethal for dog puppies in a homozygous state. Survival may be possible for some time with intense medical care. Dogs with a heterozygous genotype (T/delT) are heterozygous carriers and can pass on the mutation by a chance of 50% to their progeny. In contrast, dogs not having a mutation in the FGA gene, in particular not having the mutation NC_006597.3:g.52240694deIA (SEQ ID NO: 1: FGA:g.6296delT, SEQ ID NO: 2: FGA:g.1734deIT), can be regarded as healthy dogs, thus, representing healthy dogs with respect to afibrinogenemia.

As described herein, reference to the fibrinogen alpha chain (FGA) gene means the FGA gene located on the canine chromosome 15 (CanFam 2.0). The nucleic acid region containing the FGA gene comprises the nucleic acid sequence of bp 53,204,069 to bp 67,208,667 (14,004.6 kb) on the canine chromosome 15 (CanFam 2.0).

In the present disclosure, sequence information is obtained from the region containing the FGA gene comprises the nucleic acid sequence of bp 53,204,069 to bp 67,208,667 (14,004.6 kb) on the canine chromosome 15 (CanFam 2.0) to determine a mutation in the FGA gene on at least one allele of the FGA gene, wherein the mutation is responsible that the dog is affected from afibrinogenemia or will develop afibrinogenemia.

Accordingly, the present disclosure provides a method for assessing the risk of a canine mammal to develop a bleeding disorder related to a variant of the fibrinogen alpha chain (FGA) gene, the method comprising determining the presence or deletion located in said FGA gene at a nucleotide position corresponding to position 6296 of SEQ ID NO: 1 (CanFam 3.1) or position 1734 of SEQ ID NO: 2 (CanFam 3.1), wherein the deletion at said nucleotide position indicates an increased risk for the canine mammal of developing said bleeding disorder. In various embodiments, the nucleotide is deleted in both alleles of the FGA gene. The presence of the wild-type nucleotide at the nucleotide position corresponding to position 6296 of SEQ ID NO: 1 (CanFam 3.1), or position 1734 of SEQ ID NO: 2 (CanFam 3.1) indicates that the canine mammal will not be developing said bleeding disorder. In various embodiments, the wild-typ nucleotide is present in both alleles of the FGA gene. The said method for assessing the risk of a canine mammal to develop a bleeding disorder related to a variant of the FGA gene may also be a method for assessing susceptibility of a canine mammal for a bleeding disorder related to a variant of the FGA gene.

In one aspect, the method for assessing the risk of a canine mammal to develop a bleeding disorder related to a variant of the FGA gene is for diagnosing a canine mammal for said bleeding disorder, or for a predisposition to such a bleeding disorder, wherein the deletion of the nucleotide at the nucleotide position corresponding to position 6296 of SEQ ID NO: 1 or position 1734 of SEQ ID NO: 2 indicates that the canine mammal is affected from or is suffering from said bleeding disorder, or has a predisposition to said bleeding disorder. Accordingly, the present disclosure provides a method for diagnosing a canine mammal for a bleeding disorder related to a variant of the FGA gene, or for a predisposition to such a bleeding disorder, the method comprising determining the presence or or deletion of a nucleotide located in said FGA gene at a nucleotide position corresponding to position 6296 of SEQ ID NO: 1 or position 1734 of SEQ ID NO: 2, wherein the deletion of the nucleotide at the nucleotide position corresponding to position 6296 of SEQ ID NO: 1 or position 1734 of SEQ ID NO: 2 indicates that the canine mammal is affected from or is suffering from said bleeding disorder, or has a predisposition to said bleeding disorder. In various embodiments, the nucleotide absent in both alleles of the FGA gene. The said method for diagnosing a canine mammal may also be a method for diagnosing a canine mammal for susceptibility of a bleeding disorder related to a variant of the FGA gene.

In another aspect, the method for assessing the risk of a canine mammal to develop a bleeding disorder related to a variant of the FGA gene is for identifying and/or selecting a homozygous unaffected canine mammal with respect to a bleeding disorder-related variant of the FGA gene, wherein the presence of the wild-type nucleotide in both alleles of the FGA gene at the nucleotide position corresponding to position 6296 of SEQ ID NO: 1 or position 1734 of SEQ ID NO: 2 indicates that the canine mammal is homozygous unaffected with respect to said bleeding disorder-related variant of the FGA gene. Accordingly, the present disclosure provides a method for identifying and/or selecting a homozygous unaffected canine mammal with respect to a bleeding disorder-related variant of the FGA gene, the method comprising determining the presence or deletion of the nucleotide located in said FGA gene at a nucleotide position corresponding to position 6296 of SEQ ID NO: 1 or position 1734 of SEQ ID NO: 2, wherein the presence of the said nucleotide in both alleles of the FGA gene at the nucleotide position corresponding to position 6296 of SEQ ID NO: 1 or position 1734 of SEQ ID NO: 2 indicates that the canine mammal is homozygous unaffected with respect to said bleeding disorder-related variant of the FGA gene.

In still another aspect, the method for assessing the risk of a canine mammal to develop a bleeding disorder related to a variant of the FGA gene is for identifying and/or selecting a heterozygous canine mammal with respect to a bleeding disorder-related variant of the FGA gene, wherein the deletion of the nucleotide the nucleotide position corresponding to position 6296 of SEQ ID NO: 1 or position 1734 of SEQ ID NO: 2 indicates that the canine mammal is heterozygous with respect to said bleeding disorder-related variant of the FGA gene. Accordingly, the present disclosure provides a method for identifying and/or selecting a heterozygous canine mammal with respect to a bleeding disorder-related variant of the FGA gene, the method comprising determining the presence or deletion of the nucleotide located in said FGA gene at a nucleotide position corresponding to position 6296 of SEQ ID NO: 1 or position 1734 of SEQ ID NO: 2, wherein the deletion of said nucleotide in one allele at the nucleotide position corresponding to position 6296 of SEQ ID NO: 1 or position 1734 of SEQ ID NO: 2 indicates that the canine mammal is heterozygous with respect to said bleeding disorder-related variant of the FGA gene. In various embodiments, the nucleotide is deleted in both alleles of the FGA gene.

The homozygous unaffected canine mammals identified and/or selected according to the methods of the present disclosure can be used for breeding. Likewise, the methods for identifying and/or selecting homozygous unaffected and/or heterozygous canine mammals are useful for avoiding mating among canine mammals, which are heterozygous with respect to a bleeding disorder-related variant of the FGA gene. Furthermore, the methods for identifying and/or selecting homozygous unaffected and/or heterozygous canine mammals are useful for avoiding mating among homozygous unaffected canine mammals and heterozygous canine mammals. Accordingly, also disclosed herein but not claimed is breeding a canine mammal comprising identifying and/or selecting a canine mammal, which is homozygous unaffected with respect to a bleeding disorder-related variant of the fibrinogen alpha chain (FGA) gene, comprising determining the presence or absence of a genetic marker located in said FGA gene at a nucleotide position corresponding to position 6296 of SEQ ID NO: 1 or position 1734 of SEQ ID NO: 2, wherein the presence of the genetic marker in both alleles of the FGA gene at said nucleotide position indicates that the canine mammal is homozygous unaffected with respect to said bleeding disorder-related variant of the canine FGA gene. The breeding may further comprise identifying and/or selecting a canine mammal, which is heterozygous with respect to a bleeding disorder-related variant of the FGA gene, wherein the absence of the genetic marker in one allele at said nucleotide position indicates that the canine mammal is heterozygous with respect to said bleeding disorder-related variant of the canine FGA gene. Further disclosed herein but not claimed is breeding a canine mammal comprising identifying and/or selecting a canine mammal, which is heterozygous with respect to a bleeding disorder-related variant of the FGA gene, comprising determining the presence or absence of a genetic marker located in said FGA gene at a nucleotide position corresponding to position 6296 of SEQ ID NO: 1 or position 1734 of SEQ ID NO: 2, wherein the absence of the genetic marker in one allele at said nucleotide position indicates that the canine mammal is heterozygous with respect to said bleeding disorder-related variant of the canine FGA gene. In various embodiments, the genetic marker is absent in both alleles of the FGA gene.

The canine mammal according to the present disclosure encompasses, without being limited thereto, fox-like canids which include, *e.g.,* the kit fox *(Vulpes aelox)* and the red fox *(Vulpes vulpes),* and wolf-like canids (genus *Canis, Cuon,* and *Lycaon)* which include, e.g., the dog *(Canis lupus familiaris,* also known as *Canis familiaris),* gray wolf *(Canis lupus),* eastern wolf *(Canis lycaon),* golden jackal *(Canis aureus),* dhole *(Cuon alpinus),* and African wild dog *(Lycaon pictus).*

In the present disclosure, the canine mammal preferably is a dog or a breed of dogs belonging to the species *Canis (lupus) familiaris.* More preferably, the dog or breed of dogs is any one of a Dachshund, Chihuahua, Bichon Frise, St. Bernard, Borzoi, Vizsla or American Collie. In particularly preferred embodiments, the dog or breed of dogs is a Dachshund. The Dachshund may be, *e.g.,* a smooth-haired Dachshund, a wire-haired Dachshund, or a long-haired Dachshund. Specific Dachshunds encompassed by the present disclosure, without being limited thereto, are miniature Dachshunds and rabbit Dachshunds. Preferably, the miniature Dachshund is a wire-haired Dachshund, and the rabbit Dachshund preferably is a long-haired rabbit Dachshund.

In various embodiments of the present disclosure, the bleeding disorder is hemophilia. Preferably, the hemophilia is Factor I (fibrinogen) deficiency. Preferably, the Factor I (fibrinogen) deficiency is afibrinogenemia (sometimes also referred to as hypofibrinogenemia). Thus, in preferred embodiments of the present disclosure, the bleeding disorder is Factor I (fibrinogen) deficiency. In particularly preferred embodiments of the present disclosure, the bleeding disorder is afibrinogenemia (or hypofibrinogenemia).

Afibrinogenemia is sometimes called congenital (inherited) afibrinogenemia. Therefore, both terms may be used herein interchangeably.

Homozygosity mapping among two affected Dachshund showed a consensus region at 53,204,069-67,208,667 bp (14,004.6 kb) containing the candidate genes fibrinogen alpha chain (FGA), fibrinogen beta chain (FGB) and fibrinogen gamma chain (FGG) on dog chromosome 15 (CanFam2.0). The corresponding region on CanFam3.1 is located at 50,188,932-64,187,680 bp. Further homozygosity regions did not contain candidate genes for afibrinogenemia. The search for further potential candidate genes included beta-fibrinogenase mucrofibrase-3 precursor on CFA1, fibrinogen C domain-containing protein 1 precursor on CFA9, fibrinogen-like protein 1 precursor on CFA16, fibrinogen-like 2 L homeolog precursor on CFA18, fibrinogen silencer-binding protein on CFA29, ficolin (collagen/fibrinogen domain containing) 1 precursor on CFA18, ficolin (collagen/fibrinogen domain containing) 3 precursor on CFA9, ficolin-1 precursor, ficolin-2 precursor and ficolin-2 isoform b precursor on CFA9, ficolin-3 isoform 2 precursor and ficolin-3 isoform 2 precursor on CFA2.

An across-breed genome-wide association study confirmed the said region as strongly associated with the afibrinogenemia phenotype of the affected Dachshunds.

Sanger sequencing of the genomic DNA including all exons and their adjacent regions of all three candidate genes FGA, FGB and FGG was performed in the two affected dogs and two unaffected controls. We detected 11 single nucleotide variants (SNVs) and 5 insertion/deletion mutations (Indels) in FGA, one SNV in FGB and five SNVs and one Indel in FGG. Only four SNVs (FGA:g.2512C>A, FGA:g.2839A>G, FGA:g.5678G>T, FGA:g.6299C>T) and one Indel (FGA:g.6296deIT) was exclusively found in the two affected Dachshunds. Validation of these five mutations (FGA:g.2512C>A, FGA:g.2839A>G, FGA:g.5678G>T, FGA:g.6299C>T, FGA:g.6296deIT) revealed only FGA:g.6296delT (NC_006597.3:g.52240694delA) in perfect co-segregation with the afibrinogenemia phenotype. Bioinformatic analysis of this Indel predicted a frameshift resulting into a protein shortened by 306 amino acids (aa) (SEQ ID NOs: 3/4 and 5/6). For the protein sequence of the transcript FGA201 (SEQ ID NO: 6), an exchange of an amino acid (aa) at position 486 (p.lle486MetfsTer33) was predicted and a stop codon 33 aa (at aa 518) downstream of the mis-sense mutation. For the FGA202 transcript (SEQ ID NO: 4), the exchange of lie with Met was predicted at amino acid position 555 (p.Ile555MetfsTer33) leading to a stop codon at aa 587.

The detected NC_006597.3:g.52240694deIA (SEQ ID NO: 1: FGA:g.6296deIT, SEQ ID NO: 2: FGA:g.1734deIT), mutation was validated in 391 healthy Dachshunds including standard, miniature and rabbit size types as well as wire, smooth and long hair types (Table 1). In total ten Dachshunds were found to harbor a heterozygous mutant genotype whereas none of the tested dogs showed the homozygous mutant genotype. Further genotyping in 74 samples of 34 different dog breeds revealed no heterozygous or homozygous mutant genotype.

The NC_006597.3:g.52240694delA mutation is a single nucleotide polymorphism (SNP) at the position corresponding to nucleotide position 6296 of SEQ ID NO: 1 or position 1734 of SEQ ID NO: 2, respectively. Specifically, the SNP is a deletion of the nucleotide T at the position corresponding to nucleotide position 6296 of SEQ ID NO: 1 or nucleotide position 1734 of SEQ ID NO: 2, respectively. Accordingly, the present disclosure encompasses a genetic marker located in the fibrinogen alpha chain (FGA) gene on the canine chromosome 15 at a nucleotide position corresponding to position 6296 of SEQ ID NO: 1 or position 1734 of SEQ ID NO: 2. More specifically, the genetic marker is the nucleotide T at the position corresponding to nucleotide position 6296 of SEQ ID NO: 1 or nucleotide position 1734 of SEQ ID NO: 2.

The present disclosure also encompasses a variant of the fibrinogen alpha chain (FGA) gene on the canine chromosome 15, wherein the variant canine FGA gene is characterized by the absence of a genetic marker located in said (wild-type) FGA gene at a nucleotide position corresponding to position 6296 of SEQ ID NO: 1 or position 1734 of SEQ ID NO: 2.

In a preferred embodiment of the present disclosure, the mutation is the deletion NC_006597.3:g.52240694deIA (SEQ ID NO: 1: FGA:g.6296delT, SEQ ID NO: 2: FGA:g.1734detT), corresponding to a deletion of T at position 6296 of SEQ ID NO: 1 and/or a deletion of T at position 1734 of SEQ ID NO: 2.

At the amino acid level, the said nucleotide T is part of a base triplet "ATT", in which the SNP is the nucleotide T at position 3 of this base triplet: ATT. This base triplet is encoding the amino acid isoleucine (I - Ile), as shown at position 555 in SEQ ID NO: 4 (FGA202 transcript). The SNP, *i.e.,* deletion of the nucleotide T, results in a frameshift, as explained above, which is then leading to a stop codon after amino acid position 586, *i.e.,* at amino acid position 587, as reflected in SEQ ID NO: 3. The same is shown for the FGA201 transcript having the same isoleucine residue at position 486 of SEQ ID NO: 6. The SNP, *i.e.,* deletion of the nucleotide T at position 3 of the base triplet "AT**T**", results in a frameshift, which is then leading to a stop codon after amino acid position 517, *i.e*., at amino acid position 518, as reflected in SEQ ID NO: 5.

More specifically, the frameshift results on the amino acid level in appearing the amino acid residue methionine (M - Met) at position 555 (see SEQ ID NO: 3), and position 486 (see SEQ ID NO: 5) (deletion of the nucleotide **T** in AT**T** results in base triplet AT**G** coding for methionine). The isoleucine residue (base triplet AT**T**) present in the wild-type sequence (see position 555 of SEQ ID NO: 4 and position 486 of SEQ ID NO: 6) is no longer present in the mutant form. The amino acid residue isoleucine is also considered as a biomarker of the present disclosure. Accordingly, the present disclosure encompasses a biomarker located in the fibrinogen alpha chain (FGA) polypeptide encoded by the FGA gene on the canine chromosome 15 at an amino acid position corresponding to position 555 of SEQ ID NO: 4 or position 486 of SEQ ID NO: 6. More specifically, the biomarker is the amino acid residue isoleucine (I - Ile) at the position corresponding to amino acid position 555 of SEQ ID NO: 4 or amino acid position 486 of SEQ ID NO: 6.

Accordingly, the methods of the present disclosure may be performed making use of the biomarker located in the fibrinogen alpha chain (FGA) polypeptide encoded by the FGA gene on the canine chromosome 15 at an amino acid position corresponding to position 555 of SEQ ID NO: 4 or position 486 of SEQ ID NO: 6. Thus, encompassed herein is a method for assessing the risk of a canine mammal to develop a bleeding disorder related to a variant of the fibrinogen alpha chain (FGA) gene, the method comprising determining the presence or absence of a biomarker, in particular the amino acid residue isoleucine, located in a transcript of the FGA gene on the canine chromosome 15 at an amino acid position corresponding to position 555 of SEQ ID NO: 4 or position 486 of SEQ ID NO: 6, wherein the absence of the biomarker at said amino acid position indicates an increased risk for the canine mammal of developing said bleeding disorder. The presence of the biomarker at the amino acid position corresponding to position 555 of SEQ ID NO: 4 or position 486 of SEQ ID NO: 6 indicates that the canine mammal will not be developing said bleeding disorder.

The said method may be for diagnosing a canine mammal for a bleeding disorder related to a variant of the FGA gene, or for a predisposition to such a bleeding disorder, wherein the absence of the biomarker in the FGA transcript at the amino acid position corresponding to position 555 of SEQ ID NO: 4 or position 486 of SEQ ID NO: 6 indicates that the canine mammal is affected or suffering from said bleeding disorder, or has a predisposition to said bleeding disorder. Accordingly, disclosed herein is a method for diagnosing a canine mammal for a bleeding disorder related to a variant of the FGA gene, or for a predisposition to such a bleeding disorder, the method comprising determining the presence or absence of a biomarker, in particular the amino acid residue isoleucine, located in a transcript of the FGA gene on the canine chromosome 15 at an amino acid position corresponding to position 555 of SEQ ID NO: 4 or position 486 of SEQ ID NO: 6, wherein the absence of the biomarker in the FGA transcript at the amino acid position corresponding to position 555 of SEQ ID NO: 4 or position 486 of SEQ ID NO: 6 indicates that the canine mammal is affected or suffering from said bleeding disorder, or has a predisposition to said bleeding disorder.

The frameshift caused by the SNP of the present disclosure results in a shortened FGA transcript, which may be considered as variant FGA. In particular, the FGA transcript is shortened by 306 amino acid residues. See the length of the amino acid sequences of SEQ ID NO: 4 (892 amino acid residues) and SEQ ID NO: 6 (823 amino acid residues) as compared to the corresponding shortened transcripts shown in SEQ ID NO: 3 (586 amino acid residues) and SEQ ID NO: 5 (517 amino acid residues). The present disclosure encompasses such FGA transcripts resulting from the frameshift caused by the SNP of the present disclosure. The FGA transcripts of the present disclosure have a limited functionality.

In particular, the present disclosure encompasses a variant of the fibrinogen alpha chain polypeptide encoded by the FGA gene on the canine chromosome 15, wherein the variant FGA polypeptide is a shortened FGA transcript having a methionine residue at a position corresponding to amino acid position 555 of SEQ ID NO: 3 or position 486 of SEQ ID NO: 5. In particular, the variant FGA polypeptide is a transcript of an FGA gene missing the genetic marker of the present disclosure located in the wild-type FGA gene at a nucleotide position corresponding to position 6296 of SEQ ID NO: 1 or position 1734 of SEQ ID NO: 2. The variant FGA polypeptide is to be considered as a polypeptide of the present disclosure. Polypeptides of the present disclosure are further discussed herein below.

In various embodiments of the present disclosure, the genetic marker is nucleotide T located in the (wild-type) FGA gene on the canine chromosome 15 at a nucleotide position corresponding to position 6296 of SEQ ID NO: 1 or position 1734 of SEQ ID NO: 2. This nucleotide T is part of a base triplet "ATT", in which the nucleotide T is located at position 3 of this base triplet: AT**T**. This base triplet is encoding the amino acid isoleucine (I - Ile) at a position corresponding to amino acid position 555 of SEQ ID NO: 4 or amino acid position 486 of SEQ ID NO: 6.

The present disclosure encompasses the use of the genetic marker of the present disclosure for (i) determining the genetic status causing in a canine mammal a bleeding disorder related to the fibrinogen alpha chain (FGA) gene, (ii) identifying a canine mammal as being a heterozygous carrier with respect to a bleeding disorder-related variant of the fibrinogen alpha chain (FGA) gene, or (iii) diagnosing in a canine mammal a bleeding disorder related the fibrinogen alpha chain (FGA) gene.

The present disclosure also encompasses the use of the variant canine FGA gene of the present disclosure for (i) determining the genetic status causing in a canine mammal a bleeding disorder related to the fibrinogen alpha chain (FGA) gene, (ii) identifying a canine mammal as being a heterozygous carrier with respect to a bleeding disorder-related variant of the fibrinogen alpha chain (FGA) gene, or (iii) diagnosing in a canine mammal a bleeding disorder related the fibrinogen alpha chain (FGA) gene.

The present disclosure further encompasses the use of the biomarker, in particular the amino acid residue isoleucine located in a transcript of the FGA gene on the canine chromosome 15 at an amino acid position corresponding to position 555 of SEQ ID NO: 4 or position 486 of SEQ ID NO: 6, for diagnosing in a canine mammal a bleeding disorder related the fibrinogen alpha chain (FGA) gene.

In various embodiments of the present disclosure, the method for assessing the risk of a canine mammal to develop a bleeding disorder related to a variant of the FGA gene, which method comprises determining the presence or deletion of a nucleotide located in the FGA gene at a nucleotide position corresponding to position 6296 of SEQ ID NO: 1 or position 1734 of SEQ ID NO: 2, may further comprise correlating the deletion with the canine mammal's responsiveness to a therapeutic agent for treating the bleeding disorder. which is however not claimed.

Also, the disclosed but not claimed method for assessing the risk of a canine mammal to develop a bleeding disorder related to a variant of the FGA gene, which method comprises determining the presence or absence of a biomarker, in particular the amino acid residue isoleucine, located in a transcript of the FGA gene at an amino acid position corresponding to position 555 of SEQ ID NO: 4 or position 486 of SEQ ID NO: 6, may further comprise correlating the absence of the biomarker with the canine mammal's responsiveness to a therapeutic agent for treating the bleeding disorder. The said methods may further comprise administering to the canine mammal a therapeutic agent to abolish or alleviate the said bleeding disorder. Treatment of afibrinogenemia may include cryoprecipitate (a blood product containing concentrated fibrinogen and other clotting factors), fibrinogen (RiaSTAP) or plasma (the liquid portion of the blood which contains clotting factors).

The present disclosure provides a nucleic acid probe or primer or primer pair hybridizing or bind to a nucleic acid sequence of the canine fibrinogen alpha chain (FGA) gene on the canine chromosome 15 comprising the nucleotide position corresponding to position 6296 of SEQ ID NO: 1 or position 1734 of SEQ ID NO: 2. More specifically, the probes or primers actually bind to the position of the SNP as disclosed herein, namely a position corresponding to 6296 of SEQ ID NO: 1 or position 1734 of SEQ ID NO: 2. Such specific oligonucleotides in accordance with the present disclosure are typically oligonucleotides of at least 14 to 21 nucleotide bases in length designed to detect a difference of a single base in the target's genetic sequence of the canine mammal to be tested. in particular, the nucleic acid probe or primer or primer pair of the present disclosure comprises an oligonucleotide or a pair of oligonucleotides, which may have a length of about 15-50 nucleotides, preferably of about 15-30 nucleotides, more preferably of about 15-25 nucleotides, and still more preferably of about 17-23 nucleotides. The nucleic acid probe or primer or primer pair may be used in any method according to the present disclosure, and/or for identifying a heterozygous genotype with respect to a bleeding disorder-related variant of the fibrinogen alpha chain (FGA) gene on the canine chromosome 15. If the nucleic acid probe or primer or primer pair of the present disclosure is used in any method provided by the present disclosure, the hybridizing is performed under stringent conditions.

Hybridization may be performed under stringent or highly stringent conditions. "Stringent or highly stringent conditions" of hybridization are well known to or can be established by the person skilled in the art according to conventional protocols. Appropriate stringent conditions for each sequence may be established on the basis of well-known parameters such as temperature, composition of the nucleic acid molecules, salt conditions, etc.; *see,* for example, Sambrook et al., "Molecular Cloning, A Laboratory Manual", CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson. Typical (highly stringent) conditions comprise hybridization at 65°C in 0.5xSSC and 0.1% SDS or hybridization at 42°C in 50% formamide, 4xSSC and 0.1% SDS. Hybridization is usually followed by washing to remove unspecific signals. Washing conditions include conditions such as 65°C, 0.2xSSC and 0.1% SDS or 2xSSC and 0.1% SDS or 0.3xSSC and 0.1 % SDS at 25°C - 65°C.

Also disclosed herein is a nucleic acid molecule comprising a polynucleotide selected from the group consisting of: (a) a polynucleotide encoding a polypeptide having the amino acid sequence of SEQ ID NO: 3 or 5, or a fragment of said polypeptide; (b) a polynucleotide encoding the amino acid sequence of SEQ ID NO: 3 or 5; (c) a polynucleotide which is at least 90% identical to the polynucleotide of (a); (d) a polynucleotide encoding a polypeptide having an amino acid sequence that is at least 90% identical to the amino acid sequence of SEQ ID NO: 3 or 5; (e) a polynucleotide which hybridizes under stringent conditions to the polynucleotide of any one of (a) to (d); (f) a polynucleotide that is the complement of the full length of a polynucleotide of any of (a) to (e). Further disclosed herein but not claimed is a vector comprising the said nucleic acid molecule. The nucleic acid molecule may be operatively linked to one or more expression control sequences allowing expression in prokaryotic or eukaryotic cells.

Also disclosed herein but not claimed is a host cell transformed or transfected, or comprising, or genetically engineered, with the aforementioned nucleic acid molecule, or the aforementioned vector. The host cell may contain a polypeptide expressed from the said nucleic acid molecule, or expressed from the said vector.

Further disclosed herein but not claimed is a method of making a polypeptide encoded by the nucleic acid molecule of the present disclosure, comprising culturing a host cell of the present disclosure under conditions such that the polypeptide encoded by the nucleic acid molecule of the present disclosure is expressed, and recovering the polypeptide encoded by said nucleic acid molecule.

Still further, disclosed herein but not claimed is a polypeptide encoded by the nucleic acid molecule of the present disclosure, or obtainable by the method of the present disclosure.

Also disclosed herein but not claimed is an antibody or fragment thereof that binds specifically to a polypeptide of the present disclosure. The antibody may be specifically binding to a variant of the FGA polypeptide, which variant FGA polypeptide is a shortened FGA transcript resulting from the frameshift caused by the SNP of the present disclosure.

Further disclosed herein but not claimed is a composition, in particular a diagnostic composition, comprising (i) the nucleic acid molecule of the present disclosure, (ii) the vector of the present disclosure, (iii) the host cell of the present disclosure, (iv) the polypeptide of the present disclosure, or (v) the antibody of the present disclosure.

Further disclosed herein but not claimed is a method of diagnosing a canine mammal for a bleeding disorder related to a variant of the fibrinogen alpha chain (FGA) gene, the method comprising determining in a sample obtained from the canine mammal a polypeptide of the present disclosure. In particular, the method may comprise determining in a sample obtained from the canine mammal a variant of the FGA polypeptide, which variant FGA polypeptide is a shortened FGA transcript resulting from the frameshift caused by the SNP of the present disclosure.

Also disclosed herein but not claimed is a method of diagnosing a canine mammal for a bleeding disorder related to a variant of the FGA gene, the method comprising the use of an antibody of the present disclosure. The method may comprises the use of an antibody specifically binding to a variant of the FGA polypeptide, which variant FGA polypeptide is a shortened FGA transcript resulting from the frameshift caused by the SNP of the present disclosure.

Further disclosed herein but not claimed is a kit comprising at least one container and at least one oligonucleotide, wherein said oligonucleotide is capable of detecting the presence or absence of a genetic marker of the present disclosure, in particular a genetic marker located in the canine FGA gene at a nucleotide position corresponding to position 6296 of SEQ ID NO: 1 or position 1734 of SEQ ID NO: 2. the The at least one oligonucleotide may be at least one nucleic acid probe or primer or primer pair of the present disclosure described elsewhere herein. The oligonucleotide may have a length of about 15-50 nucleotides, preferably of about 15-30 nucleotides, more preferably of about 15-25 nucleotides, and still more preferably of about 17-23 nucleotides. The at least one oligonucleotide may be stored in the at least one container of the kit. The kit may optionally further comprise an instruction manual with regard to the use of the kit in any of the methods disclosed herein. Accordingly, disclosed herein is the use of the kit of the present disclosure in a method of the present disclosure. The kit of the present disclosure may contains at least one antibody of the present disclosure. In particular, the kit may comprise an antibody or fragment thereof that binds specifically to a variant of the FGA polypeptide, which variant FGA polypeptide is a shortened FGA transcript resulting from the frameshift caused by the SNP of the present disclosure.

The methods of the present disclosure are not practiced on the body of the canine mammal, but encompass one or more samples obtained from the canine mammal. Thus, the methods of the present disclosure can be considered as *in vitro* methods. Accordingly, in the methods of the present disclosure, determining the presence or absence of a genetic marker located in the FGA gene at the nucleotide position corresponding to position 6296 of SEQ ID NO: 1 or position 1734 of SEQ ID NO: 2 encompasses determining the presence or absence of the genetic marker in a sample obtained from the canine mammal. The sample is a sample suitable for genetic testing, in particular for determining genetic markers like single nucleotide polymorphisms (SNPs). Thus, the sample is a sample containing, or suspected to contain, DNA of the canine mammal. In this context, the term "sample" may refer to any material containing nucleated cells from a canine mammal to be tested. Preferably, the methods of the present disclosure, which comprise determining the presence or deletion of the nucleotide located in the FGA gene at the nucleotide position corresponding to position 6296 of SEQ ID NO: 1 or position 1734 of SEQ ID NO: 2, are practiced on DNA samples, *i.e.,* samples obtained from the canine mammal which have been processed to purify and/or isolate the canine mammal's DNA. Such a sample may be considered a DNA sample.

Likewise, the methods of the present disclosure, which comprise determining the presence or absence of a biomarker, in particular the amino acid residue isoleucine, located in a transcript of the FGA gene on the canine chromosome 15 at an amino acid position corresponding to position 555 of SEQ ID NO: 4 or position 486 of SEQ ID NO: 6, encompass determining the presence or absence of a biomarker in a sample obtained from the canine mammal. The sample is a sample suitable for testing for the biomarker, in particular for determining the amino acid residue isoleucine in a transcript of the FGA gene at an amino acid position corresponding to position 555 of SEQ ID NO: 4 or position 486 of SEQ ID NO: 6. Thus, the sample is a sample containing, or suspected to contain, a transcript of the canine FGA or a variant thereof, in particular a shortened FGA transcript. The methods of the present disclosure, which comprise determining the presence or absence of a biomarker, in particular the amino acid residue isoleucine, located in a transcript of the FGA gene on the canine chromosome 15 at an amino acid position corresponding to position 555 of SEQ ID NO: 4 or position 486 of SEQ ID NO: 6, may be practiced on samples that contain or are suspected to contain, FGA transcripts. The samples obtained from the canine mammal may be processed to purify and/or isolate FGA transcripts of the present disclosure, which are suspected to be present in the sample obtained from the canine mammal. Such a sample may be considered as an FGA transcript sample.

In the present disclosure, the terms "sample" or "biological sample" may be used interchangeably. The sample or biological sample to be used in the methods of the present disclosure may be any one of a blood sample, sputum, hair roots, milk, sperm, body fluids as well as tissues including nucleated cells. The sample obtained from the canine mammal may be a blood sample, hair sample, or sputum.

Any of the methods disclosed herein may comprise a separate step of providing a sample obtained from the canine mammal.

DNA extraction, isolation and purification methods are well-known in the art and can be applied in the present disclosure. Standard protocols for the isolation of genomic DNA are, *inter alia,* referred to in Sambrook, J., Russell. D. W.. Molecular Cloning: A Laboratory Manual, the third edition, Cold Spring Harbor Laboratory Press, Cold Spring Harbor. New York, 1.31-1.38, 2001, and Sharma, R.C., et al. "A rapid procedure for isolation of RNA-free genomic DNA from mammalian cells", BioTechniques, 14:176-178, 1993.

According to the present disclosure, the term "SNP" refers to a single nucleotide polymorphism at a particular position in the genome of a canine mammal that varies among a population of individuals. SNPs can be identified by their location within the disclosed particular sequence, *i.e.,* within the nucleic acid region of 53,204,069 to 67,208,667 (14,004.6 kb) on the canine chromosome 15 (CanFam 2.0). The SNP identified as being useful in the methods of the present disclosure is particularly reflected by the variant NC_006597.3:g.52240694deIA corresponding to a deletion of T at position 6296 (6296T -> 6296deIT) of SEQ ID NO: 1 or a deletion of T at position 1734 (1734T -> 1734deIT) of SEQ ID NO: 2).

The term "determining (or detecting) the presence or absence of the genetic marker in DNA of the canine mammal" in accordance with the present disclosure refers to a method for determining or identifying whether a particular nucleotide sequence is present in a DNA sample. There are several methods known by those skilled in the art for determining whether such nucleotide sequence is present in a DNA sample. Thus, any of the methods disclosed herein may comprise, e.g., PCR, ligase chain reaction, restriction digestion, direct sequencing, nucleic acid amplification techniques, hybridization techniques and/or immunoassays. More specifically, methods known by those skilled in the art include the amplification of a DNA segment encompassing the genetic marker by means of the polymerase chain reaction (PCR) or any other amplification method, interrogate the genetic marker by means of allele-specific hybridization, the 3'-exonuclease assay (Taqman assay), fluorescent dye and quenching agent-based PCR assay, the use of allele-specific restriction enzymes (RFLP-based techniques), direct sequencing, the oligonucleotide ligation assay (OLA), pyrosequencing, the invader assay, minisequencing, DHPLC-based techniques, single strand conformational polymorphism (SSCP), allele-specific PCR, denaturating gradient gel electrophoresis (DGGE), temperature gradient gel electrophoresis (TGGE), chemical mismatch cleavage (CMC), heteroduplex analysis based system, techniques based on mass spectroscopy, invasive cleavage assay, polymorphism ratio sequencing (PRS), microarrays, a rolling circle extension assay, HPLC-based techniques, extension based assays, ARMS (Amplification Refractory Mutation System), ALEX (Amplification Refractory Mutation Linear Extension), SBCE (Single base chain extension), molecular beacon assays, invader (Third wave technologies), ligase chain reaction assays, 5'-nuclease assay-based techniques, hybridization capillary array electrophoresis (CAE), protein truncation assays (PTT), immunoassays, and solid phase hybridization (dot blot, reverse dot blot, chips). This list of methods is not meant to be exclusive, but just to illustrate the diversity of available methods. Some of these methods can be performed in accordance with the methods of the present disclosure in microarray format (microchips) or on beads.

### EXAMPLES

The following examples are included to demonstrate preferred embodiments of the disclosure. It should be appreciated by those of skill in the art that the techniques disclosed in the examples that follow represent techniques discovered by the inventors to function well in the practice of the invention, and thus can be considered to constitute preferred modes for its practice.

### Example 1

### Phenotype

Two seven months old Dachshunds affected with severe bleeding tendency were investigated. The dogs were littermates from miniature wire-haired Dachshund parents. As none of the parents or the other littermates showed signs of the disease, an autosomal recessive mode of inheritance was assumed. Clinical examination of the affected dogs confirmed the diagnosis of afibrinogenemia.

### Homozygosity mapping and genome-wide-association study

Homozygosity mapping among two affected Dachshund showed a consensus region at 53,204,069-67,208,667 bp (14,004.6 kb) containing the candidate genes fibrinogen alpha chain (FGA), fibrinogen beta chain (FGB) and fibrinogen gamma chain (FGG) on dog chromosome 15 (CanFam2.0). The corresponding region on CanFam3.1 is located at 50,188,932-64,187,680 bp. Further homozygosity regions did not contain candidate genes for afibrinogenemia. The search for further potential candidate genes included beta-fibrinogenase mucrofibrase-3 precursor on CFA1, fibrinogen C domain-containing protein 1 precursor on CFA9, fibrinogen-like protein 1 precursor on CFA16, fibrinogen-like 2 L homeolog precursor on CFA18, fibrinogen silencer-binding protein on CFA29, ficolin (collagen/fibrinogen domain containing) 1 precursor on CFA18, ficolin (collagen/fibrinogen domain containing) 3 precursor on CFA9, ficolin-1 precursor, ficolin-2 precursor and ficolin-2 isoform b precursor on CFA9, ficolin-3 isoform 2 precursor and ficolin-3 isoform 2 precursor on CFA2.

An across-breed genome-wide association study confirmed this region as strongly associated with the phenotype of the affected Dachshunds.

### Sequencing

Sanger sequencing of the genomic DNA including all exons and their adjacent regions of all three candidate genes FGA, FGB and FGG was performed in the two affected dogs and two unaffected controls. We detected 11 single nucleotide variants (SNVs) and 5 insertion/deletion mutations (Indels) in FGA, one SNV in FGB and five SNVs and one Indel in FGG. Only four SNVs (FGA:g.2512C>A, FGA:g.2839A>G, FGA:g.5678G>T, FGA:g.6299C>T) and one Indel (FGA:g.6296deIT) were exclusively found in the two affected Dachshund. Validation of these five mutations (FGA:g.2512C>A, FGA:g.2839A>G, FGA:g.5678G>T, FGA:g.6299C>T, FGA:g.6296deIT) revealed only FGA:g.6296deIT (NC_006597.3:g.52240694delA) in perfect co-segregation with the afibrinogenemia phenotype. Bioinformatic analysis of this Indel predicted a frameshift resulting into a protein shortened by 306 amino acids (aa) (SEQ ID No. 3/4 and 5/6). For the protein sequence of the transcript FGA201, an exchange of an amino acid (aa) at position 486 (p.Ile486MetfsTer33) was predicted and a stop codon 33 aa (at aa 518) downstream of the mis-sense mutation. For the FGA202 transcript, the exchange of lie with Met was predicted at amino acid position 555 (p.Ile555MetfsTer33) leading to a stop codon at aa 587.

### Validation

The detected NC_006597.3:g.52240694deIA (SEQ ID NO: 1: FGA:g.6296deIT, SEQ ID NO: 2. FGA:g.1734deIT), mutation was validated in 391 healthy Dachshunds including standard, miniature and rabbit size types as well as wire, smooth and long hair types (Table 1). In total ten Dachshunds were found to harbor a heterozygous mutant genotype whereas none of the tested dogs showed the homozygous mutant genotype. Further genotyping in 74 samples of 34 different dog breeds revealed no heterozygous or homozygous mutant genotype.

### Example 2

### Animals

Two afibrinogenemia-affected Dachshunds and 14 related dogs were investigated for their health status using a questionnaire and by interrogation with the dog owners. The affected dogs were further clinically examined at the age of seven weeks and investigated for their capillary bleeding time. In addition, 377 Dachshunds and 74 samples of 32 other breeds were used as controls.

### Homozygosity mapping and genome-wide association analysis

Both afibrinogenemia-affected Dachshunds were genotyped on the canine Illumina high density beadchip (Illumina) for 173,662 SNPs. Data were quality controlled for Hardy-Weinberg equilibrium (P-value<0.0000001), minor allele frequencies (MAF) of >0.05 and a genotyping rate per SNP and animal of >0.95. Homozygosity mapping was performed using PLINK, version 1.07 (http://pngu.mgh.harvard.edu/purcell/plink/) and all SNPs after quality control for genotyping rate per SNP and animal. A total of 169,877 SNPs has been employed for homozygosity mapping. Across-breed association analysis was performed using PLINK, version 1.07, and a total of 168,071 SNPs.

### Sequencing

All exons and exon/intron boundaries in the candidate genes FGA, FGB and FGG were sequenced in two afibrinogenemia-affected Dachshunds and two unaffected control Dachshunds using 3500 Genetic Analyzer for Sanger-sequencing (Thermo Fisher Scientific, Waltham, Massachusetts, USA). Primers were designed using Primer3 (http://frodo.wi.mit.edu/primer3/) and PCR-reactions were run for 95°C for 4 minutes, 38 cycles of 94°C for 30 seconds, primer specific annealing temperature for 30 seconds and 72°C for 45 seconds.

Gene models were confirmed using cDNA sequencing of one unaffected control dog. RNA was purified using RNeasy Mini Kit (Qiagen, MD, USA) and transcribe into cDNA using the Maxima First Strand cDNA Synthesis Kit (Fermentas, Thermo Fisher Scientific, Waltham, MA, USA).

### Genotyping of candidate variants

The detected variant NC_006597.3:g.52240694deIA (SEQ ID NO: 1: FGA:g.6296delT, SEQ ID NO: 2. FGA:g.1734deIT), was genotyped in 391 Dachshunds and further 74 samples of different breeds using a restriction fragment length polymorphism (Table 2-5). According to manufacturer's protocols reactions were prepared with the digestion enzyme MIuCl and CutSmart buffer derived from NEB (NEB BioLabs, Ipswich, MA, USA). Variant effect prediction was performed using the Variant Effect Predictor (McLaren *et al.* 2010) with the dog reference genome CanFam3.1 for SIFT (Kumar *et al.* 2009) predictions and PolyPhen-2 (Adzhubei *et al.* 2010).

### Tables

Table 1. Validation of the NC_006597.3:g.52240694delA (SEQ ID NO 1: FGA:g.6296deIT, SEQ ID NO: 2. FGA:g.1734delT) mutation in Dachshunds.

| Breed | N | T/T | T/delT | delT/delT |
|---|---|---|---|---|
| Afibrinogenemia-affected Dachshund | 2 | 0 | 0 | 2 |
| Dachshund- smooth-haired | 22 | 22 | 0 | 0 |
| Dachshund - wire-haired | 211 | 209 | 2 | 0 |
| Dachshund - long-haired | 17 | 17 | 0 | 0 |
| Miniature Dachshund - smooth-haired | 11 | 11 | 0 | 0 |
| Miniature Dachshund - wire-haired | 75 | 67 | 8 | 0 |
| Miniature Dachshund - long-haired | 18 | 18 | 0 | 0 |
| Rabbit Dachshund - smooth-haired | 8 | 8 | 0 | 0 |
| Rabbit Dachshund - wire-haired | 25 | 25 | 0 | 0 |
| Rabbit Dachshund - long-haired | 4 | 4 | 0 | 0 |

**Table 2. Primer sequences, annealing temperature and product size is shown for validation of the NC_006597.3:g.52240694deIA (SEQ ID NO: 1: FGA:g.6296deIT, SEQ ID NO: 2. FGA:g.1734delT) mutation.**

| Primer forward | Primer reverse | Annealing temp. (°C) | Product size (bp) |
|---|---|---|---|
| TTGGAGCTCTGGAAGTTCTG | CTTTAGCATGGCCTCTTTTG | 58 | 704 |

**Table 3. PCR-recipe for validation of the NC_006597.3:g.52240694deIA (SEQ ID NO: 1: FGA:g.6296delT, SEQ ID NO: 2. FGA:g.1734delT) mutation.**

| Reagents | 1 sample (µl) |
|---|---|
| H2O | 19.3 |
| Buffer | 3.1 |
| Enhancer | 6.2 |
| dNTPs | 0.6 |
| Primer forward | 1.3 |
| Primer reverse | 1.3 |
| Taq-Polymerase | 0.12 |
| | Total volume: 30 µl/sample |

**Table 4. Master mix for digestion of the NC_006597.3:g.52240694deIA (SEQ ID NO: 1: FGA:g.6296delT, SEQ ID NO: 2. FGA:g.1734deIT) mutation containing amplicon to be used for the PCR-based Restriction Fragment Length Polymorphism (RFLP)**

| Reagents | 1 sample (µl) |
|---|---|
| H2O | 8.25 |
| 1X CutSmart^{®} Buffer | 1.5 |
| PCR-product | 5 |
| MIuCl enzyme | 0.25 |
| | total volume: 15µl/sample |

**Table 5. Restriction fragment patterns of the PCR-based RFLP for the NC_006597.3:g.52240694deIA (SEQ ID NO: 1: FGA:g.6296delT, SEQ ID NO: 2. FGA:g.1734delT) mutation.**

| Genotype | Fragments | Result |
|---|---|---|
| T/T | 411, 149, 144 | homozygous wild type |
| T/delT | 559, 411, 149, 144 | heterozygous |
| delT/delT | 559, 144 | homozygous mutant |

All of the methods disclosed and claimed herein can be made and executed by the skilled practitioner in light of the present disclosure without undue experimentation.

### REFERENCES

Adzhubei I.A., Schmidt S., Peshkin L., Ramensky V.E., Gerasimova A., Bork P., Kondrashov A.S. & Sunyaev S.R. (2010) A method and server for predicting damaging missense mutations. Nature Methods 7, 248-9.
Brooks M. (1999a) Hereditary bleeding disorders in dogs and cats. Veterinary medicine 94, 555-65.
Brooks M. (1999b) A review of canine inherited bleeding disorders: biochemical and molecular strategies for disease characterization and carrier detection. Journal of Heredity 90, 112-8.
Chambers G. (2013) Treatment of afibrinogenemia in a chihuahua. Journal of the American Animal Hospital Association 49, 70-4.
de Moerloose P., Casini A. & Neerman-Arbez M. (2013) Congenital fibrinogen disorders: an update. Seminars in Thrombosis and Hemostasis 39, 585-95.
Kammermann B., Gmür J. & Stünzi H. (1971) Afibrinogenämie beim Hund. Zentralblatt für Veterinärmedizin Reihe A 18, 192-205.
Kumar P., Henikoff S. & Ng P.C. (2009) Predicting the effects of coding non-synonymous variants on protein function using the SIFT algorithm. Nature Protocols 4, 1073-81.
Lak M., Keihani M., Elahi F., Peyvandi F. & Mannucci P.M. (1999) Bleeding and thrombosis in 55 patients with inherited afibrinogenaemia. British Journal of Haematology 107, 204-6.
Lippi G., Pasalic L. & Favaloro E.J. (2015) Detection of mild inherited disorders of blood coagulation: current options and personal recommendations. Expert Review of Hematology 8, 527-42.
Mammen E.F. (1976) Congenital dysfibrinogenemias: molecular abnormalities of fibrinogen. Blut 33, 229-34.
McLaren W., Pritchard B., Rios D., Chen Y., Flicek P. & Cunningham F. (2010) Deriving the consequences of genomic variants with the Ensembl API and SNP Effect Predictor. Bioinformatics 26, 2069-70.
Metzger J., Pfahler S. & Distl O. (2016) Variant detection and runs of homozygosity in next generation sequencing data elucidate the genetic background of Lundehund syndrome. BMC Genomics 17, 535.
Neerman-Arbez M. & de Moerloose P. (2007) Mutations in the fibrinogen gene cluster accounting for congenital afibrinogenemia: an update and report of 10 novel mutations. Human Mutation 28, 540-53.
Neerman-Arbez M., de Moerloose P., Honsberger A., Parlier G., Arnuti B., Biron C., Borg J.Y., Eber S., Meili E., Peter-Salonen K., Ripoll L., Vervel C., d'Oiron R., Staeger P., Antonarakis S.E. & Morris M.A. (2001) Molecular analysis of the fibrinogen gene cluster in 16 patients with congenital afibrinogenemia: novel truncating mutations in the FGA and FGG genes. Human Genetics 108, 237-40.
Smith J.W., Day T.K., Andrew Mackin B. & MVS D. (2005) Diagnosing bleeding disorders. Compendium on continuing education for the practicing veterinarian.
Wilkerson M.J., Johnson G.S., Stockham S. & Riley L. (2005) Afibrinogenemia and a circulating antibody against fibrinogen in a Bichon Frise dog. Vet Clin Pathol 34, 148-55.

## Claims

1. A method for assessing the risk of a canine mammal to develop a bleeding disorder related to a variant of the fibrinogen alpha chain (FGA) gene, the method comprising determining the presence or deletion of the nucleotide located in the FGA gene at a nucleotide position corresponding to position 6296 of SEQ ID NO: 1 or position 1734 of SEQ ID NO: 2, wherein the deletion of the nucleotide at said nucleotide position indicates an increased risk for the canine mammal of developing said bleeding disorder.

2. The method of claim 1, wherein the presence of the nucleotide at the nucleotide position corresponding to position 6296 of SEQ ID NO: 1 or position 1734 of SEQ ID NO: 2 indicates that the canine mammal will not be developing said bleeding disorder.

3. The method of claim 1, which is for diagnosing a canine mammal for a bleeding disorder related to a variant of the FGA gene, or a predisposition to such a bleeding disorder, wherein the deletion of the nucleotide at the nucleotide position corresponding to position 6296 of SEQ ID NO: 1 or position 1734 of SEQ ID NO: 2 indicates that the canine mammal is affected or suffering from said bleeding disorder, or has a predisposition to said bleeding disorder.

4. The method of claim 1, which is for identifying and/or selecting a homozygous unaffected canine mammal with respect to a bleeding disorder-related variant of the FGA gene, wherein the presence of the nucleotide in both alleles of the FGA gene at the nucleotide position corresponding to position 6296 of SEQ ID NO: 1 or position 1734 of SEQ ID NO: 2 indicates that the canine mammal is homozygous unaffected with respect to said bleeding disorder-related variant of the FGA gene.

5. The method of claim 1, which is for identifying and/or selecting a heterozygous canine mammal with respect to a bleeding disorder-related variant of the FGA gene, wherein the deletion of the nucleotide in one allele at the nucleotide position corresponding to position 6296 of SEQ ID NO: 1 or position 1734 of SEQ ID NO: 2 indicates that the canine mammal is heterozygous with respect to said bleeding disorder-related variant of the FGA gene.

6. A method for identifying and/or selecting a canine mammal, which is homozygous unaffected with respect to a bleeding disorder-related variant of the fibrinogen alpha chain (FGA) gene, the method comprising determining the presence or deletion of the nucleotide located in the FGA gene at a nucleotide position corresponding to position 6296 of SEQ ID NO: 1 or position 1734 of SEQ ID NO: 2, wherein the presence of the nucleotide in both alleles of the FGA gene at said nucleotide position indicates that the canine mammal is homozygous unaffected with respect to said bleeding disorder-related variant of the FGA gene.

7. The method of claim 6, further comprising identifying and/or selecting a canine mammal, which is heterozygous with respect to a bleeding disorder-related variant of the FGA gene, wherein the deletion of the nucleotide in one allele at said nucleotide position indicates that the canine mammal is heterozygous with respect to said bleeding disorder-related variant of the FGA gene.

8. The method of any one of claims 1 and 3 wherein said nucleotide is deleted in one or both alleles of the FGA gene.

9. The method of claim 2, wherein said nucleotide is present in both alleles of the FGA gene.

10. The method of any one of claims 1 to 9, wherein the canine mammal is a dog or a breed of dogs belonging to the species *Canis familiaris,* preferably wherein the dog or breed of dogs is any one of a Dachshund, Chihuahua, Bichon Frise, St. Bernard, Borzoi, Vizsla or American Collie, more preferably the dog or breed of dogs is a Dachshund.

11. The method of any one of claims 1 to 10, wherein the bleeding disorder is hemophilia, preferably wherein the bleeding disorder is Factor I (fibrinogen) deficiency, more preferably wherein the Factor I (fibrinogen) deficiency is afibrinogenemia or hypofibrinogenemia.

12. A nucleic acid probe or primer hybridizing to a nucleic acid sequence of the canine fibrinogen alpha chain (FGA) gene on canine chromosome 15 comprising a nucleotide located in the fibrinogen alpha chain (FGA) gene on the canine chromosome 15 at a nucleotide position corresponding to position 6296 of SEQ ID NO: 1 or position 1734 of SEQ ID NO: 2, wherein the nucleic acid probe or primer is designed to specifically detect the presence of said nucleotide located in the FGA gene at a nucleotide position corresponding to position 6296 of SEQ ID NO: 1 or position 1734 of SEQ ID NO: 2.

13. A nucleic acid probe or primer hybridizing to a nucleic acid sequence of a variant of the fibrinogen alpha chain (FGA) gene on the canine chromosome 15, wherein the variant canine FGA gene is **characterized by** the deletion of the nucleotide located in said wild type FGA gene at a nucleotide position corresponding to position 6296 of SEQ ID NO: 1 or position 1734 of SEQ ID NO: 2, and wherein the nucleic acid probe or primer is designed to specifically detect the deletion of said nucleotide located in the FGA gene at a nucleotide position corresponding to position 6296 of SEQ ID NO: 1 or position 1734 of SEQ ID NO: 2.

14. The method of any one of claims 1 to 11, or the nucleic acid probe or primer of claim 12 or 13, wherein said nucleotide is nucleotide T located in the FGA gene on the canine chromosome 15 at a nucleotide position corresponding to position 6296 of SEQ ID NO: 1 or position 1734 of SEQ ID NO: 2.

15. Use of the nucleic acid probe or primer of claim 12 or 13 for (i) determining the genetic status causing in a canine mammal a bleeding disorder related to the fibrinogen alpha chain (FGA) gene, (ii) identifying and/or selecting a canine mammal as being a heterozygous carrier with respect to a bleeding disorder-related variant of the fibrinogen alpha chain (FGA) gene, (iii) diagnosing in a canine mammal a bleeding disorder related the fibrinogen alpha chain (FGA) gene, or (iv) evaluating an increased risk for a bleeding disorder.

## Patentansprüche

1. Verfahren zur Bewertung des Risikos eines hundeartigen Säugetieres, eine Blutungsstörung zu entwickeln, welche mit einer Variante des Fibrinogen-alpha-Ketten (FGA)-Gens zusammenhängt, wobei das Verfahren die Bestimmung des Vorhandenseins oder der Deletion eines Nukleotids umfasst, welches sich in dem FGA-Gen an einer Nukleotidposition befindet, welche der Position 6296 von SEQ ID NO: 1 oder der Position 1734 von SEQ ID NO: 2 entspricht, wobei die Deletion des Nukleotids an der Nukleotidposition ein erhöhtes Risiko für das hundeartige Säugetier anzeigt, die Blutungsstörung zu entwickeln.

2. Verfahren nach Anspruch 1, wobei das Vorhandensein des Nukleotids an der Nukleotidposition, welche der Position 6296 von SEQ ID NO: 1 oder der Position 1734 von SEQ ID NO: 2 entspricht, anzeigt, dass das hundeartige Säugetier die Blutungsstörung nicht entwickeln wird.

3. Verfahren nach Anspruch 1, welches dazu dient, bei einem hundeartigen Säugetier eine Blutungsstörung, die mit einer Variante des FGA-Gens zusammenhängt, oder eine Prädisposition für eine solche Blutungsstörung, zu diagnostizieren, wobei die Deletion des Nukleotids an der Nukleotidposition, welche der Position 6296 von SEQ ID NO: 1 oder der Position 1734 von SEQ ID NO: 2 entspricht, anzeigt, dass das hundeartige Säugetier von der Blutungsstörung betroffen ist oder darunter leidet, oder eine Prädisposition für die Blutungsstörung hat.

4. Verfahren nach Anspruch 1, welches der Identifizierung und/oder Selektion eines homozygoten, nicht betroffenen hundeartigen Säugetieres in Bezug auf eine mit einer Blutungsstörung zusammenhängende Variante des FGA-Gens dient, wobei das Vorhandensein des Nukleotids in beiden Allelen des FGA-Gens an der Nukleotidposition, welche der Position 6296 von SEQ ID NO: 1 oder der Position 1734 von SEQ ID NO: 2 entspricht, anzeigt, dass das hundeartige Säugetier in Bezug auf die mit der Blutungsstörung zusammenhängende Variante des FGA-Gens homozygot nicht betroffen ist.

5. Verfahren nach Anspruch 1, welches der Identifizierung und/oder Selektion eines heterozygoten hundeartigen Säugetieres in Bezug auf eine mit einer Blutungsstörung zusammenhängende Variante des FGA-Gens dient, wobei die Deletion des Nukleotids in einem Allel an der Nukleotidposition, welche der Position 6296 von SEQ ID NO: 1 oder der Position 1734 von SEQ ID NO: 2 entspricht, anzeigt, dass das hundeartige Säugetier in Bezug auf die mit der Blutungsstörung zusammenhängende Variante des FGA-Gens heterozygot ist.

6. Verfahren zur Identifizierung und/oder Selektion eines hundeartigen Säugetieres, welches homozygot nicht betroffen ist in Bezug auf eine mit einer Blutungsstörung zusammenhängende Variante des Fibrinogen-alpha-Ketten-Gens (FGA), wobei das Verfahren die Bestimmung des Vorhandenseins oder der Deletion des Nukleotids umfasst, welches sich in dem FGA-Gen an einer Nukleotidposition befindet, welche der Position 6296 von SEQ ID NO: 1 oder der Position 1734 von SEQ ID NO: 2 entspricht, wobei das Vorhandensein des Nukleotids in beiden Allelen des FGA-Gens an der genannten Nukleotidposition anzeigt, dass das hundeartige Säugetier homozygot nicht beeinträchtigt ist in Bezug auf die mit der Blutungsstörung zusammenhängende Variante des FGA-Gens.

7. Verfahren nach Anspruch 6, weiterhin umfassend das Identifizieren und/oder Selektieren eines hundeartigen Säugetieres, welches heterozygot in Bezug auf eine mit einer Blutungsstörung zusammenhängende Variante des FGA-Gens ist, wobei die Deletion des Nukleotids in einem Allel an der Nukleotidposition anzeigt, dass das hundeartige Säugetier heterozygot in Bezug auf die mit der Blutungsstörung zusammenhängende Variante des FGA-Gens ist.

8. Verfahren nach einem der Ansprüche 1 und 3, wobei das Nukleotid in einem oder beiden Allelen des FGA-Gens deletiert ist.

9. Verfahren nach Anspruch 2, wobei das Nukleotid in beiden Allelen des FGA-Gens vorhanden ist.

10. Verfahren nach einem der Ansprüche 1 bis 9, wobei das hundeartige Säugetier ein Hund oder eine Hunderasse ist, welche/r zur Spezies *Canis familiaris* gehört, wobei der Hund oder die Hunderasse vorzugsweise ein Dackel, Chihuahua, Bichon Frisé, Bernhardiner, Barsoi, Vizsla oder American Collie ist, noch mehr bevorzugt ist der Hund oder die Hunderasse ein Dackel.

11. Verfahren nach einem der Ansprüche 1 bis 10, wobei die Blutungsstörung Hämophilie ist, vorzugsweise wobei die Blutungsstörung Faktor I (Fibrinogen)-Mangel ist, noch mehr bevorzugt wobei der Faktor I (Fibrinogen)-Mangel Afibrinogenämie oder Hypofibrinogenämie ist.

12. Nukleinsäuresonde oder Primer, welche/r mit einer Nukleinsäuresequenz des Hundefibrinogen-alpha-Ketten (FGA)-Gens auf dem Hundechromosom 15 hybridisiert, umfassend ein Nukleotid, welches sich in dem Fibrinogen-alpha-Ketten (FGA)-Gen auf dem Hundechromosom 15 an einer Nukleotidposition befindet, welche der Position 6296 von SEQ ID NO: 1 oder der Position 1734 von SEQ ID NO: 2 entspricht, wobei die Nukleinsäuresonde oder der Primer derart gestaltet ist, spezifisch das Vorhandensein des Nukleotids zu detektieren, welches sich in dem FGA-Gen an einer Nukleotidposition befindet, welche der Position 6296 von SEQ ID NO: 1 oder der Position 1734 von SEQ ID NO: 2 entspricht.

13. Nukleinsäuresonde oder Primer, welche/r der mit einer Nukleinsäuresequenz einer Variante des Fibrinogen-alpha-Ketten (FGA)-Gens auf dem Hunde-Chromosom 15 hybridisiert, wobei die Variante des Hunde-FGA-Gens durch die Deletion eines Nukleotids gekennzeichnet ist, welches sich in dem Wildtyp FGA-Gen an einer Nukleotidposition befindet, welche der Position 6296 von SEQ ID NO: 1 oder der Position 1734 von SEQ ID NO: 2 entspricht, und wobei die Nukleinsäuresonde oder der Primer derart gestaltet ist, spezifisch die Deletion des Nukleotids zu detektieren, welches sich in dem FGA-Gen an einer Nukleotidposition befindet, welche der Position 6296 von SEQ ID NO: 1 oder der Position 1734 von SEQ ID NO: 2 entspricht.

14. Das Verfahren nach einem der Ansprüche 1 bis 11 oder die Nukleinsäuresonde oder der Primer nach Anspruch 12 oder 13, wobei es sich bei dem Nukleotid um das Nukleotid T handelt, welches sich im FGA-Gen auf dem Hundechromosom 15 an einer Nukleotidposition befindet, welche der Position 6296 von SEQ ID NO: 1 oder der Position 1734 von SEQ ID NO: 2 entspricht.

15. Verwendung der Nukleinsäuresonde oder des Primers nach Anspruch 12 oder 13 zur (i) Bestimmung des genetischen Status, welcher in einem hundeartigen Säugetier eine Blutungsstörung verursacht, welche mit dem Fibrinogen-alpha-Ketten-Gen (FGA) zusammenhängt, (ii) Identifizierung und/oder Selektion eines hundeartigen Säugetieres als heterozygoter Träger einer mit einer Blutungsstörung zusammenhängenden Variante des Fibrinogen-alpha-Kette (FGA)-Gens, (iii) Diagnose einer mit dem Fibrinogen-alpha-Kette (FGA)-Gen zusammenhängenden Blutungsstörung bei einem hundeartigen Säugetier, oder (iv) Bewertung eines erhöhten Risikos für eine Blutungsstörung.

## Revendications

1. Méthode d'évaluation du risque qu'un mammifère canin développe un trouble de la coagulation lié à un variant du gène de la chaîne alpha du fibrinogène (FGA), la méthode consistant à déterminer la présence ou la délétion d'un nucléotide situé dans le gène FGA à une position nucléotidique correspondant à la position 6296 de SEQ ID NO : 1 ou à la position 1734 de SEQ ID NO : 2, la délétion du nucléotide à ladite position nucléotidique indiquant un risque accru pour le mammifère canin de développer le trouble de la coagulation en question.

2. La méthode de la revendication 1, dans laquelle la présence du nucléotide à la position correspondant à la position 6296 de SEQ ID NO : 1 ou à la position 1734 de SEQ ID NO : 2 indique que le mammifère canin ne développera pas le trouble de la coagulation.

3. La méthode de la revendication 1, qui sert à diagnostiquer chez un mammifère canin un trouble de la coagulation lié à une variante du gène FGA, ou une prédisposition à un tel trouble de la coagulation, dans laquelle la délétion du nucléotide à la position correspondant à la position 6296 de SEQ ID NO : 1 ou à la position 1734 de SEQ ID NO : 2 indique que le mammifère canin est affecté ou souffre dudit trouble de la coagulation, ou a une prédisposition à ce trouble de la coagulation.

4. La méthode de la revendication 1, qui consiste à identifier et/ou sélectionner un mammifère canin homozygote non affecté par rapport à une variante du gène FGA liée aux troubles de la coagulation, dans laquelle la présence du nucléotide dans les deux allèles du gène FGA à la position nucléotidique correspondant à la position 6296 de SEQ ID NO : 1 ou à la position 1734 de SEQ ID NO : 2 indique que le mammifère canin est homozygote non affecté par rapport à ladite variante du gène FGA liée aux troubles de la coagulation.

5. La méthode de la revendication 1, qui consiste à identifier et/ou sélectionner un mammifère canin hétérozygote par rapport à une variante du gène FGA liée aux troubles de la coagulation, dans laquelle la délétion du nucléotide dans un allèle à la position nucléotidique correspondant à la position 6296 de SEQ ID NO : 1 ou à la position 1734 de SEQ ID NO : 2 indique que le mammifère canin est hétérozygote par rapport à ladite variante du gène FGA liée aux troubles de la coagulation.

6. Méthode d'identification et/ou de sélection d'un mammifère canin homozygote non affecté par une variante du gène de la chaîne alpha du fibrinogène (FGA) liée à un trouble de la coagulation, la méthode comprenant la détermination de la présence ou de la délétion du nucléotide situé dans le gène FGA à une position nucléotidique correspondant à la position 6296 de SEQ ID NO : 1 ou à la position 1734 de SEQ ID NO : 2, la présence du nucléotide dans les deux allèles du gène FGA à ladite position nucléotidique indiquant que le mammifère canin est homozygote non affecté en ce qui concerne ladite variante du gène FGA liée aux troubles de la coagulation.

7. La méthode de la revendication 6, comprenant en outre l'identification et/ou la sélection d'un mammifère canin qui est hétérozygote par rapport à une variante du gène FGA liée aux troubles de la coagulation, dans laquelle la délétion du nucléotide dans un allèle à ladite position nucléotidique indique que le mammifère canin est hétérozygote par rapport à ladite variante du gène FGA liée aux troubles de la coagulation.

8. La méthode de l'une des revendications 1 et 3, dans laquelle ledit nucléotide est délété d'un ou des deux allèles du gène FGA.

9. La méthode de la revendication 2, dans laquelle ledit nucléotide est présent dans les deux allèles du gène FGA.

10. La méthode de l'une des revendications 1 à 9, dans laquelle le mammifère canin est un chien ou une race de chiens appartenant à l'espèce *Canis familiaris,* de préférence dans laquelle le chien ou la race de chiens est un teckel, un chihuahua, un bichon frisé, un saint-bemard, un barzoï, un vizsla ou un colley américain, plus préférentiellement un teckel.

11. La méthode de l'une des revendications 1 à 10, dans laquelle le trouble de la coagulation est l'hémophilie, de préférence dans laquelle le trouble de la coagulation est la déficience en facteur I (fibrinogène), plus préférentiellement dans laquelle la déficience en facteur I (fibrinogène) est l'afibrinogénémie ou l'hypofibrinogénémie.

12. Sonde ou amorce d'acide nucléique s'hybridant à une séquence d'acide nucléique du gène de la chaîne alpha du fibrinogène canin (FGA) sur le chromosome 15 canin, comprenant un nucléotide situé dans le gène de la chaîne alpha du fibrinogène (FGA) sur le chromosome 15 canin à une position nucléotidique correspondant à la position 6296 de SEQ ID NO : 1 ou à la position 1734 de SEQ ID NO : 2, dans lequel la sonde ou l'amorce d'acide nucléique est conçue pour détecter spécifiquement la présence dudit nucléotide situé dans le gène FGA à une position nucléotidique correspondant à la position 6296 de SEQ ID NO : 1 ou à la position 1734 de SEQ ID NO : 2.

13. Sonde d'acide nucléique ou amorce s'hybridant à une séquence d'acide nucléique d'une variante du gène de la chaîne alpha du fibrinogène (FGA) sur le chromosome 15 du chien, dans laquelle la variante du gène FGA du chien est **caractérisée par** la délétion d'un nucléotide situé dans ledit gène FGA (de type sauvage) à une position nucléotidique correspondant à la position 6296 de SEQ ID NO : 1 ou à la position 1734 de SEQ ID NO : 2, et dans lequel la sonde ou l'amorce d'acide nucléique est conçue pour détecter spécifiquement la délétion dudit nucléotide situé dans le gène FGA à une position nucléotidique correspondant à la position 6296 de SEQ ID NO : 1 ou à la position 1734 de SEQ ID NO : 2.

14. La méthode de l'une des revendications 1 à 11, ou la sonde ou l'amorce d'acide nucléique de la revendication 12 ou 13, dans laquelle ledit nucléotide est le nucléotide T situé dans le gène FGA sur le chromosome 15 canin à une position nucléotidique correspondant à la position 6296 de SEQ ID NO : 1 ou à la position 1734 de SEQ ID NO : 2.

15. Utilisation de la sonde ou de l'amorce d'acide nucléique de la revendication 12 ou 13 pour (i) déterminer le statut génétique provoquant chez un mammifère canin un trouble de la coagulation lié au gène de la chaîne alpha du fibrinogène (FGA), (ii) identifier et/ou sélectionner un mammifère canin comme porteur hétérozygote d'une variante du gène de la chaîne alpha du fibrinogène (FGA) liée à un trouble de la coagulation, (iii) diagnostiquer chez un mammifère canin un trouble de la coagulation lié au gène de la chaîne alpha du fibrinogène (FGA), ou (iv) évaluer un risque accru de trouble de la coagulation.
